# EUROPÄISCHE PATENTSCHRIFT

(11) **EP 3 424 983 B1**
(45) Veröffentlichungstag und Bekanntmachung des Hinweises auf die Patenterteilung: **23.10.2019**
(21) Anmeldenummer: 17180115.2
(22) Anmeldetag: 06.07.2017
(51) Int. Cl.: C08G 77/28, C08K 9/06, C08L 83/08

(54) **POLYMERWERKSTOFFE MIT SILANBASIERENDEN ÜBERTRAGUNGSREAGENZIEN**
POLYMERIC MATERIALS WITH SILANE-BASED TRANSMISSION REAGENTS
POLYMÈRE À ÉMULSION DE L'AGENT DE TRANSFERT À BASE DE SILANE

(43) Veröffentlichungstag der Anmeldung: 09.01.2019
(73) Patentinhaber: Ivoclar Vivadent AG, 9494 Schaan (LI); Technische Universität Wien, 1040 Wien (AT)
(72) Erfinder: Moszner, Norbert, 9493 Mauren (LI); Angermann, Jörg, 7320 Sargans (CH); Fischer, Urs Karl, 9320 Arbon (CH); Lamparth, Iris, 9472 Grabs (CH); Catel, Yohann, 9470 Buchs (CH); Liska, Robert, 2123 Schleinbach (AT)
(74) Vertreter: Uexküll & Stolberg

(56) Entgegenhaltungen:
- DE-A1-102011 050 672

## Beschreibung

Die vorliegende Erfindung betrifft radikalisch polymerisierbare Silane, die als solche, in Form von Polysiloxan-Kondensaten oder in füllstoffgebundener Form als Kettenüberträger zur Kontrolle und Steuerung der Netzwerkstruktur bei der radikalischen Polymerisation geeignet sind.

Radikalische Polymerisate werden durch radikalische Polymerisation von einem (Homopolymerisate) oder mehreren (Copolymerisate) radikalisch polymerisierbaren Monomeren gebildet. Dabei werden je nach der Funktionalität der polymerisierten Monomere lineare (bei monofunktionellen Monomeren) oder vernetzte (bei di- oder multifunktionellen Monomeren) Polymere erhalten. Ein großer Vorteil der radikalischen Polymerisationen besteht darin, dass sich einerseits viele technisch relevante Monomere (u.a. Ethylen, Vinylmonomere, wie Styrol, Vinylchlorid, Acrylnitril und Vinylacetat, Diene, (Meth)acrylate und (Meth)acrylamide) radikalisch polymerisieren lassen und sich andererseits die radikalische Polymerisation in Substanz (Bulkpolymerisation), Lösung, Suspension oder Emulsion durchführen lässt. Zur Polymerisationsauslösung werden radikalbildende Initiatoren zugesetzt, die durch Thermolyse, Photolyse oder Redoxreaktion Radikale bilden.

Die radikalische Polymerisation läuft nach einem Kettenwachstumsmechanismus ab, bei dem sich die aus dem Initiator gebildeten polymerisationsauslösenden Primärradikale an die Doppelbindung der Monomere addieren. Die so gebildeten Startradikale addieren in einer schnellen Wachstumsreaktion viele weitere Monomermoleküle, bis das Wachstum der Polymerradikale durch Kombination oder Disproportionierung abgebrochen wird und die fertigen Makromoleküle entstehen.

Durch die Zugabe von Kettenüberträgern, sogenannten Reglern, lässt sich die zahlenmittlere Molmasse des gebildeten Polymers gezielt regulieren (vgl. H. G. Elias, Makromoleküle, Bd. 1, 6. Aufl., Wiley-VCH, Weinheim etc. 199, 299-352). Zu den bekannten Kettenüberträgern zählen beispielsweise die Mercaptane, die durch Übertragung eines Wasserstoffatoms Thiyl-Radikale bilden, die dann eine neue Polymerisationskette initiieren.

Als Kettenüberträger haben sich doppelbindungshaltige Reagenzien besonders bewährt, die nach einem radikalischen Additions-Fragmentierungs-Kettenübertragungs-Mechanismus (engl.: "**a**ddition-**f**ragmentation **c**hain **t**ransfer", AFCT) reagieren. Als AFCT-Reagenzien sind Schwefelverbindungen, wie Allylsulfide, Allylsulfone, Dithioester, Dithiocarbamate, Xanthate und Trithiocarbonate besonders wirksam und gut untersucht (Moad et al., Polymer 49, 1079-1131 (2008)). Darüber hinaus sind reversible AFCT-Reagenzien (RAFT-Reagenzien), wie z.B. Dithioester, Dithiocarbamate, Trithiocarbonate oder Xanthate, aus der kontrollierten radikalischen Polymerisation bekannt (vgl. z.B. Moad et al., aaO)

Die US 2,694,699 beschreibt die Homo- und Copolymerisation von α-Sulfonyloxyacrylaten. Als Kettenregler können Alkylmercaptane zugesetzt werden.

Die US 5,932,675 offenbart ein Verfahren zur Herstellung von Polymeren mit geringem Molekulargewicht durch radikalische Polymerisation, bei dem die Kontrolle des Molekulargewichts durch die Zugabe z.B. von α-(t-Butanthiomethyl)styrol als Kettenübertragungsreagenz erfolgt.

Die EP 2 965 741 A1 offenbart radikalisch polymerisierbare Dentalwerkstoffe, die Allylsulfone als Kettenübertragungsreagenz enthalten. Die Werkstoffe zeichnen sich durch eine geringe Polymerisationsschrumpfungsspannung aus.

Die EP 3 090 722 A1 betrifft radikalisch polymerisierbare Dentalwerkstoffe, die Vinylsulfonsäureester als AFCT-Reagenz enthalten. Die Werkstoffe weisen einen engen Glasübergangsbereich auf und bilden homogene Polymernetzwerke.

Bei den bekannten AFTC-Reagenzien handelt es sich in der Regel um niedermolekulare Verbindungen, die bei unvollständiger Polymerisation aus dem gehärteten Material entweichen können. Insbesondere können sie durch Speichel aus gehärteten Dentalwerkstoffen aufgewaschen werden, was unter toxikologischen Gesichtspunkten unerwünscht ist.

DE102011050672 offenbart ein Kieselsäure(hetero)polykondensat, umfassend (Meth)Acrylreste und entweder Sulfonat- oder Sulfatgruppen, die jeweils unmittelbar oder mittelbar über eine unsubstituierte oder substituierte Kohlenwasserstoffgruppe mit einer C-Si-Bindung an einem Siliciumatom gebunden sind.

Der Erfindung liegt die Aufgabe zugrunde, Stoffe bereitzustellen, die sich als Kettenübertragungsreagenzien für die radikalische Reaktion eignen, die nach der Polymerisation nicht aus dem gehärteten Material entweichen und die insbesondere nicht durch Speichel ausgewaschen werden.

Diese Aufgabe wird erfindungsgemäß durch Silanen der allgemeinen Formel I, durch Polysiloxan-Kondensate, die durch hydrolytische Kondensation dieser Silane erhältlich sind, oder durch Füllstoffe gelöst, die mit Silanen der allgemeinen Formel I oberflächenmodifiziert sind: 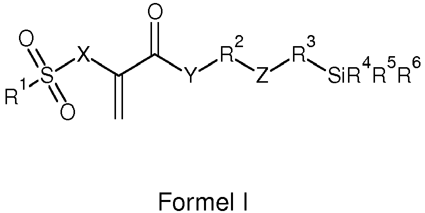 wobei
- R¹: ein linearer oder verzweigter aliphatischer C₁-C₉-Alkyl-Rest, Phenyl- oder alkylierter Phenyl-Rest ist,
- R², R³: unabhängig voneinander jeweils entfallen oder ein linearer oder verzweigter aliphatischer C₁-C₂₀-Alkylen-Rest sind, der durch S- oder O-Atome unterbrochen sein kann,
- R⁴, R⁵, R⁶: unabhängig voneinander jeweils -Cl, -O-CH₃, -O-C₂H₅, -CH₃ oder -C₂H₅ sind,
- X: CH₂ oder O ist,
- Y: entfällt, O oder NR', wobei R' H oder ein C₁₋₅-Alkylrest ist, und
- Z: entfällt, O, NR", -CO-O-, -CO-NR"-, -O-CO-O-, -O-CO-NR"-, oder -NR"-CO-NR"- ist, wobei R" H oder ein C₁₋₅-Alkylrest ist.

Die Reste R² und R³ können nicht gleichzeitig entfallen und wenn R² oder R³ entfällt, entfällt vorzugsweise auch Z.

Unter einem alkylierten Phenyl-Rest wird ein Phenyl-Rest verstanden, der durch 1 bis 5 Alkylgruppen, vorzugsweise durch 1 Alkylgruppe substituiert ist. Bevorzugt sind C₁₋₁₀-Alkyl- und insbesondere C₁₋₅-Alkylgruppen.

Bevorzugt sind Verbindungen der Formel I, in denen die Variablen die folgenden Bedeutungen haben:
- R¹: -CH₃, -C₂H₅, Phenyl oder Tolyl (H₃C-Ph-), insbesondere p-Tolyl,
- R², R³: unabhängig voneinander jeweils entfallen oder ein linearer aliphatischer C₁-C₁₀-Alkylen-Rest, der durch O-Atome unterbrochen sein kann,
- R⁴, R⁵, R⁶: unabhängig voneinander jeweils -O-CH₃ oder -O-C₂H₅,
- X: CH₂ oder O,
- Y: entfällt oder O, und
- Z: entfällt, O, -CO-O-, -CO-NH-, -O-CO-O- oder -O-CO-NH-.

Besonders bevorzugt sind Silane der Formel I, in denen die Variablen die folgenden Bedeutungen haben:
- R¹: -CH₃, -C₂H₅, Phenyl oder Tolyl (H₃C-Ph-), insbesondere p-Tolyl,
- R²: ein linearer aliphatischer C₂-C₈-Alkylen-Rest, der durch O-Atome unterbrochen sein kann,
- R³: entfällt oder ein linearer aliphatischer C₁-C₆-Alkylen-Rest,
- R⁴, R⁵, R⁶: unabhängig voneinander jeweils -O-CH₃ oder -O-C₂H₅,
- X: CH₂ oder O,
- Y: O, und
- Z: entfällt, -CO-NH- oder -O-CO-NH-.

Die Reste R⁴, R⁵ und R⁶ haben in allen Fällen vorzugsweise die gleiche Bedeutung.

Die Formel I erstreckt sich nur auf solche Verbindungen, die mit der chemischen Valenzlehre vereinbar sind. Der Hinweis, dass ein Rest durch ein oder mehrere O-Atome oder S-Atome unterbrochen ist, ist so zu verstehen, dass diese Atome jeweils in die Kohlenstoffkette des Restes eingeschoben werden. Diese Atome sind damit beidseitig durch C-Atome begrenzt und können nicht endständig sein. C₁-Reste können nicht unterbrochen sein.

Die Verbindungen der Formel I sind als solche bei der radikalischen Polymerisation als Kettenüberträger aktiv und deren Verwendung als Kettenregler ist ebenfalls Gegenstand der Erfindung. Vorzugsweise werden sie in Form von Polykondensaten oder in an Füllstoff gebundener Form eingesetzt. Wenn im Folgenden von Verbindungen der Formel I gesprochen wird, sind damit auch die Polysiloxan-Kondensate und die an Füllstoff gebundenen Silane gemeint.

Polymerisationsübertragungsaktive Si-Verbindungen der Formel I sind teilweise bekannt und lassen sich nach bekannten Synthesemethoden einfach herstellen. So lassen sich allylsulfongruppenhaltige Derivate (X = CH₂) durch Addition von lod-Verbindungen an ungesättigte Derivate (A) und nachfolgend HI-Abspaltung (B) herstellen. Anschließend kann dann durch Umsetzung mit einem geeigneten Silan (C) eine erfindungsgemäße polymerisationsübertragungsaktive Si-Verbindung der Formel I erhalten werden, wobei bei Vor- oder Zwischenstufen gegebenenfalls die Schutzgruppentechnik anzuwenden ist (Z' = O oder NR):

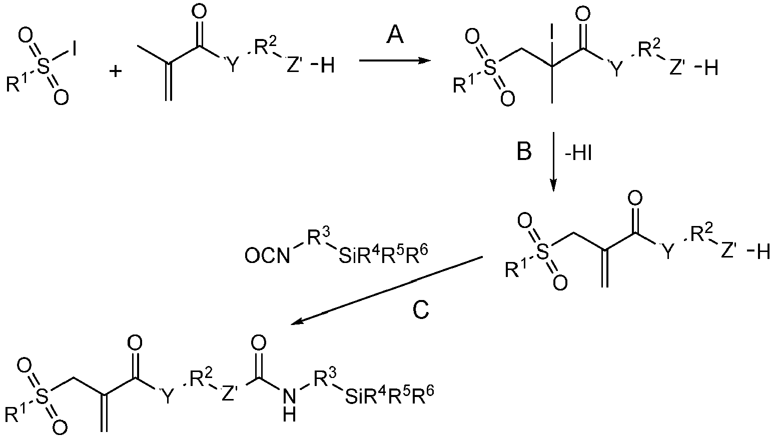

Ein konkretes Beispiel ist:

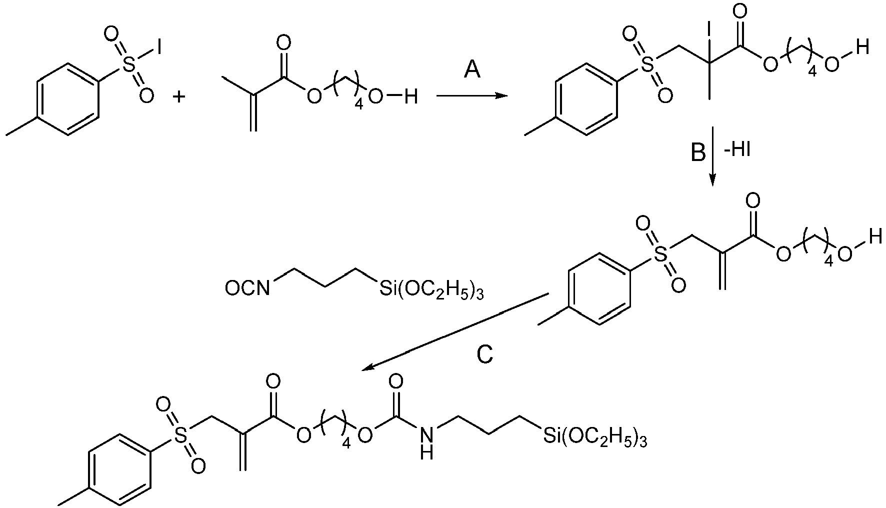

Vinylsulfonestergruppenhaltige Derivate (X = O) lassen sich durch Umsetzung von Brenztraubensäurederivaten mit Sulfonyl-Halogeniden unter basischen Bedingungen herstellen. Geht man dabei von z.B. Brenztraubensäureestern aus, die eine geschützte (G) OH-Gruppe -OG (G ist z.B. Tetrahydro-2H-pyran: THP) enthalten (A), kann nach der Vinylsulfonestersynthese die Schutzgruppe abgespalten (B) werden, und durch weitere Umsetzung mit einem geeigneten Silan (C) wird eine erfindungsgemäße polymerisationsübertragungsaktive Si-Verbindung der Formel I erhalten (Z' = O oder NR):

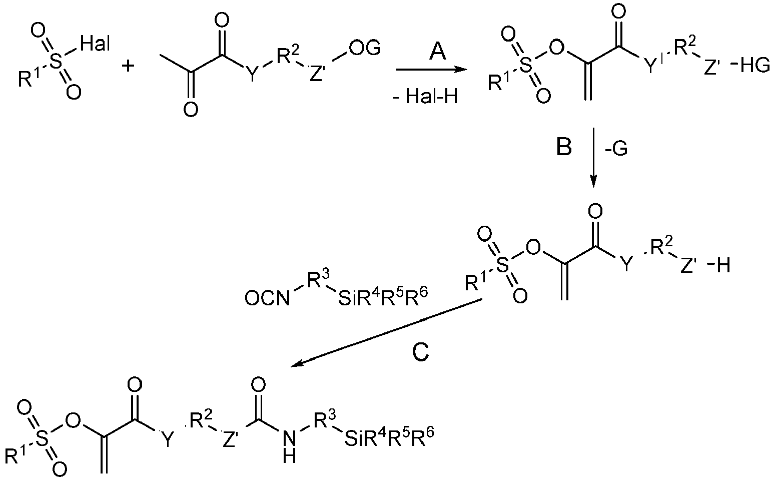

Ein konkretes Beispiel ist:

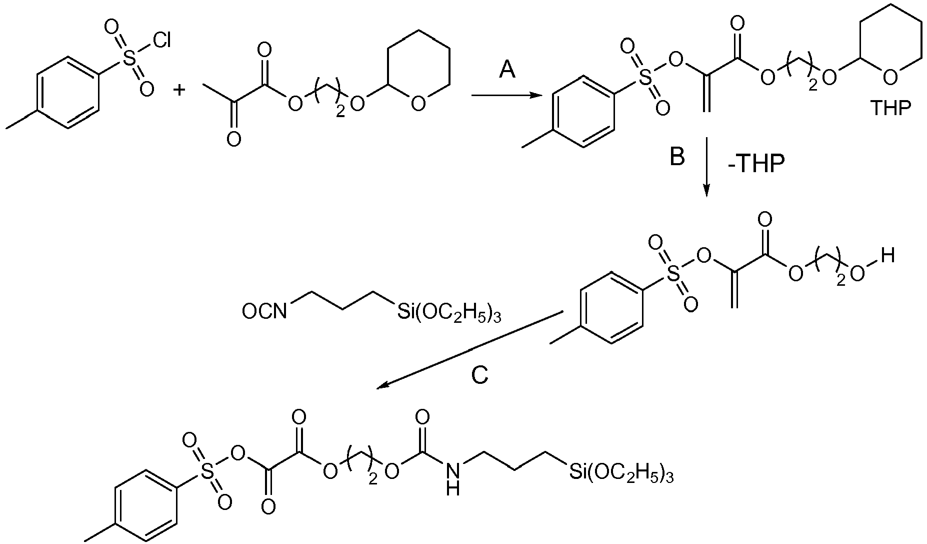

Bevorzugte Beispiele für erfindungsgemäße polymerisationsübertragungsaktiven Si-Verbindungen der Formel I sind:

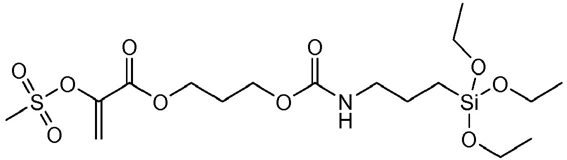

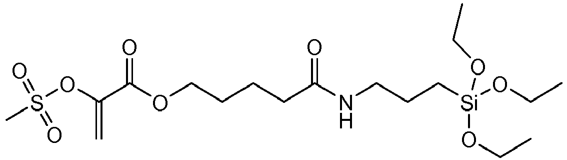

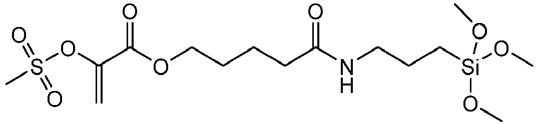

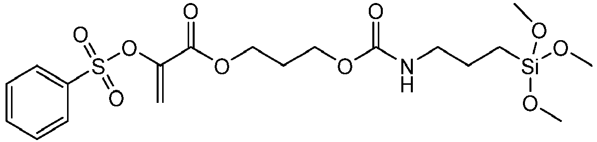

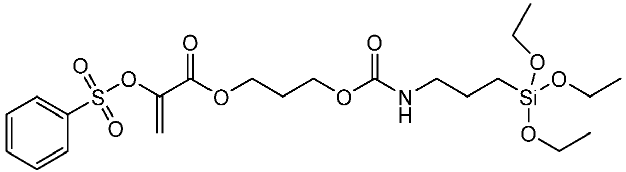

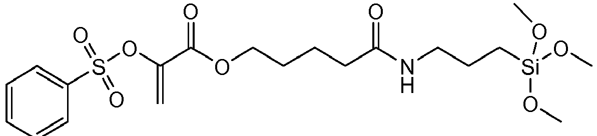

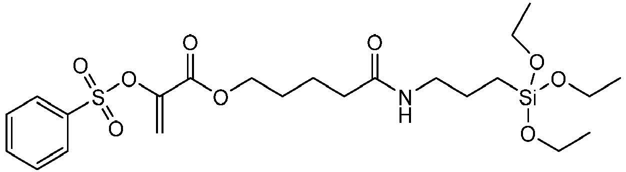

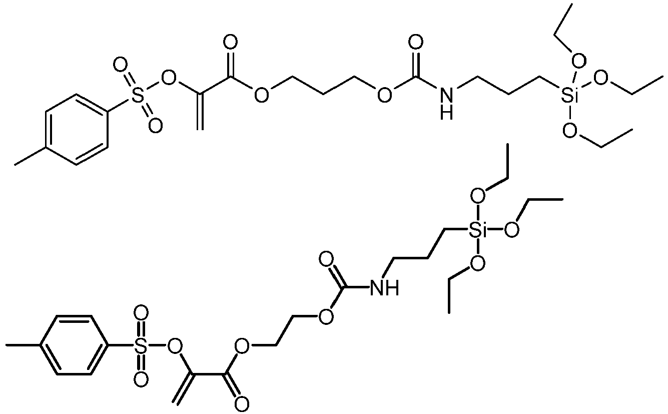

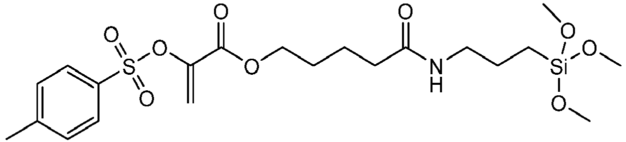

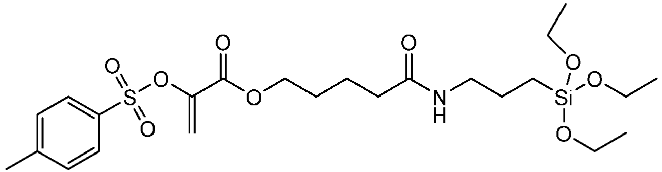

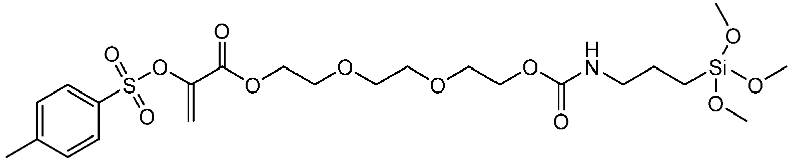

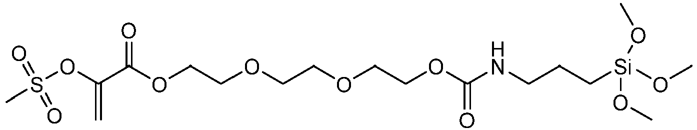

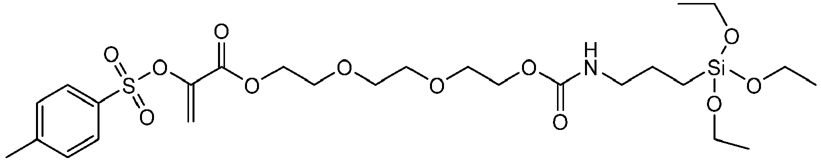

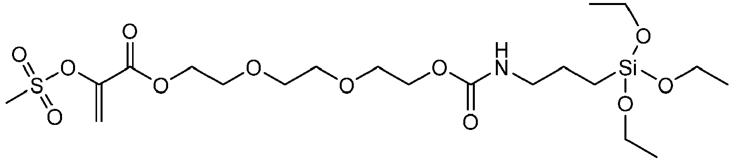

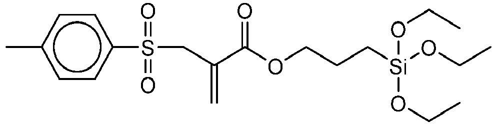

Aus den erfindungsgemäßen polymerisationsübertragungsaktiven Si-Verbindungen der Formel I lassen sich nach den aus der Sol-Gel-Chemie (vgl. Monographie: Sol-Gel-Science, C. J. Brinker, G. W. Scherer, Academic Press Inc., Boston etc. 1990) bekannten Methoden Polysiloxan-Kondensate herstellen, die praktisch in Wasser keine Löslichkeit besitzen. Vorzugsweise werden die Kondensate durch hydrolytische Kondensation hergestellt.

Bei der hydrolytischen Kondensation werden ein oder mehrere Silane der Formel I, vorzugsweise ein Silan der Formel I, in Substanz oder vorzugsweise als Lösung in einem organischen Lösungsmittel mit einer stöchiometrischen Menge oder einem Überschuss an Wasser (vorzugsweise > 1 bis 10 mol Wasser pro mol hydrolysierbarer Gruppen des Silans) versetzt und reagieren gelassen. Bevorzugte Lösungsmittel sind Aceton, Ethanol, Methanol, Isopropanol, Essigsäureethylester, Methylisobutylketon, DMF, THF, Dioxan oder eine Mischung davon. Bei Lösungen wird das Silan vorzugsweise mit dem 0,1 bis 5-fachen Volumen an Lösungsmittel verdünnt. Die Zugabe von Wasser erfolgt in einer Portion oder portionsweise, wobei das Wasser vor der Zugabe vorzugsweise mit einem organischen Lösungsmittel verdünnt wird. Hierzu sind ebenfalls die zuvor genannten Lösungsmittel bevorzugt.

Die hydrolytische Kondensation erfolgt vorzugsweise bei 0 bis 30 °C oder bei dem Siedepunkt des eingesetzten Lösungsmittels (vorzugsweise 30 bis 140°C). Gemäß einer bevorzugten Ausführungsform des Verfahrens erfolgt die Umsetzung in zwei Schritten. Hierzu werden die Komponenten zunächst bei 0 bis 30 °C gemischt, vorzugsweise bei Raumtemperatur (20°C), und dann zur Vervollständigung der Reaktion auf eine erhöhte Temperatur, vorzugsweise auf 30 bis 140°C, erwärmt. Anschließend wird das Reaktionsgemisch vorzugsweise über einen Zeitraum von 1 bis 120 h weiter gerührt, vorzugsweise bei 20 bis 140°C. Bei Alkoxysilanen erfolgt die Reaktion von Methoxysilanen schneller als die Reaktion der Ethoxysilane.

Zur Reaktionsbeschleunigung kann vorteilhaft ein geeigneter Katalysator, wie z.B. eine Säure, z.B. Essigsäure oder Salzsäure, oder eine Base, z.B. Ammoniak, ein Amin, NaOH, Methylimidazol, oder Ammoniumfluorid zugegeben werden. Der Katalysator wird vorzugsweise in einer Menge von 0,001 bis 3,0 Gew.-% bezogen auf die Silanmenge eingesetzt.

Nach Abschluss der Reaktion werden das Lösungsmittel und flüchtige Bestandteile entfernt, vorzugweise durch Abdampfen im Vakuum. Vorzugsweise werden das Lösungsmittel und flüchtige Bestandteile erst im Wasserstrahlvakuum weitgehend abgedampft und der Rückstand dann im Ölpumpenvakuum weiter getrocknet.

Der Kondensationsgrad der nach Entfernung des Lösungsmittels und flüchtiger Bestandteile erhaltenen organofunktionellen Polysiloxane kann mittels ²⁹Si-NMR-Spektroskopie ermittelt werden. In Abhängigkeit vom Kondensationsgrad und der Struktur der erfindungsgemäßen Silane werden flüssige oder feste Polykondensate erhalten. Die Reste R⁴ bis R⁶ bestimmen die Struktur der gebildeten Kondensate. Danach ist zu unterscheiden in hydrolysierbare Reste (-Cl, -O-CH₃, und -O-C₂H₅) und nicht hydrolysierbare Reste (-CH₃ und -C₂H₅). Sind die Reste R⁴ bis R⁶ alle hydrolysierbar, ergibt sich bei vollständigem Umsatz ein vernetztes Kondensat (Polysiloxan), das sich durch die folgende Formel beschreiben lässt:

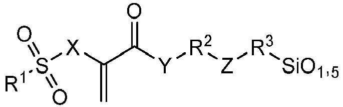

Sind nur zwei Reste, z.B. R⁴ und R⁵, hydrolysierbar, werden Polysiloxane erhalten, die sich durch die folgende Formel beschreiben lassen:

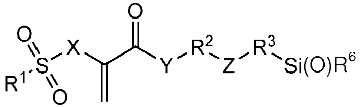

Hierbei handelt es sich um lineare Kondensate.

Wenn nur ein Rest, z.B. R⁴, hydrolysierbar ist, werden Disiloxane gemäß der folgenden Formel erhalten:

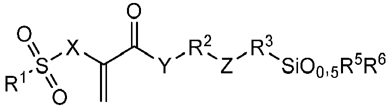

Neben der Herstellung von Polysiloxanen eignen sich erfindungsgemäße Si-Verbindungen der Formel I, die mindestens eine hydrolysierbare Gruppe R⁴ bis R⁶ enthalten, auch zur Oberflächenmodifizierung von Füllstoffen. Die Modifizierung der Füllstoffe kann auf an sich bekannte Weise erfolgen (vgl. z.B. Monographie: Silane Coupling Agents, E. P. Pluedemann, 2.Ed. Plenum Press, New York-London 1991).

Als Füllstoffe eignen sich bevorzugt anorganische partikuläre Füllstoffe, wie Pulver von Quarz und radioopaken Barium- oder Strontium-Aluminiumsilikat-Gläsern, vorzugsweise mit einer mittleren Partikelgröße von 0,01 bis 15 µm, oder röntgenopake Füllstoffe, wie Ytterbiumtrifluorid. Bevorzugte anorganische partikuläre Füllstoffe sind auch amorphe kugelförmige nanopartikuläre Füllstoffe auf der Basis von Oxiden, wie pyrogene Kieselsäure, Fällungskieselsäure, ZrO₂, ZnO, TiO₂ oder Mischoxide aus SiO₂, ZrO₂ und/oder TiO₂, vorzugsweise mit einem mittleren Partikelgröße von 10 bis 1000 nm. Bevorzugte Füllstoffe sind weiterhin partikuläres, vorzugsweise nanopartikuläres, Tantal(V)-oxid oder Bariumsulfat sowie Mischoxide von SiO₂ mit Ytterbium(III)-oxid oder Tantal(V)-oxid. Darüber hinaus können auch faserförmige Füllstoffe wie Nanofasern, Glasfasern, Polyamid- oder Kohlenstoff-Fasern eingesetzt werden. Besonders bevorzugte Füllstoffe sind Barium- oder Strontium-Aluminiumsilikat-Gläser, Mischoxide aus SiO₂ und ZrO₂, Ytterbiumfluorid.

Silane der Formel I mit mindestens einem hydrolysierbaren Rest R⁴ bis R⁶ reagieren über ihren Silanrest mit OH-Gruppen auf oxidischen Füllstoffoberflächen und werden so an den Füllstoff gebunden. Für eine optimale Oberflächenmodifizierung wird der Füllstoff vorzugsweise in einem organischen Lösungsmittel, besonders bevorzugt Ethanol, Aceton, Isopropanol, Methanol, Essigsäureethylester, THF oder eine Mischung davon, dispergiert, mit einem oder mehreren Silanen der Formel I, vorzugsweise mit einem Silan der Formel I, und Wasser (vorzugsweise 1 bis 10 mol bezogen auf die eingesetzte Silanmenge) und vorzugsweise auch mit einem Katalysator (vorzugsweise 0,001 bis 2 mol-% bezogen auf die eingesetzte Silanmenge) versetzt und gerührt. Die Reaktion wird vorzugsweise bei einer Temperatur von 0°C bis 100°C, beispielsweise bei 0°C bis Raumtemperatur (20°C) oder bei erhöhter Temperatur (30 bis 100 °C) durchgeführt. Analog zur Herstellung der Kondensate wird das Wasser vor der Zugabe bevorzugt mit Lösungsmittel verdünnt, wobei hierzu die vorgenannten Lösungsmittel bevorzugt sind. Bevorzugte Katalysatoren sind Säuren, z.B. Essigsäure oder Salzsäure, Basen, z.B. Ammoniak oder Amine, oder Ammoniumfluorid. Die Reaktionsmischung wird bis zum Abschluss der Reaktion, vorzugsweise für 1 bis 20 Stunden, z.B. über Nacht (ca. 15 Stunden), gerührt. Anschließend wird der oberflächenmodifizierte Füllstoff abgetrennt, vorzugsweise abfiltriert, dann vorzugsweise mit reinem Lösungsmittel gewaschen und anschließend bis zur Gewichtskonstanz getrocknet. Der Oberflächenmodifizierungsgrad kann aus dem Glührückstand oder einer elementaranalytischen Kohlenstoffbestimmung ermittelt werden.

Die Silane der Formel I, die daraus erhaltenen Polysiloxan-Kondensate und die damit oberflächenmodifizierte Füllstoffe verfügen über polymerisationsübertragungsaktive Allylsulfon- oder Vinylsulfonestergruppen und lassen sich zur Kontrolle und Steuerung der Netzwerkstruktur bei der Polymerisation von radikalisch polymerisierbaren Monomeren und insbesondere von multifunktionellen (Meth)acrylaten, Mischungen davon oder von Mischungen von multifunktionellen (Meth)acrylaten mit Mono(meth)acrylaten einsetzen.

Erfindungsgemäß wurde gefunden, dass die Silane der Formel I selbst dann eine wirksame Kontrolle der Polymerisation erlauben, wenn sie an Füllstoff gebunden sind oder als Kondensat vorliegen. Dies ist insoweit überraschend, weil durch die Einbindung der Reglermoleküle in größere Einheiten deren Beweglichkeit im Reaktionsmedium stark eingeschränkt wird.

Die polymerisationsübertragungsaktiven Gruppen in Verbindungen der Formel I ergeben Polymere mit einem engeren Glasübergang, d.h. der Glasübergang findet in einem engeren Temperaturbereich statt. Außerdem werden homogenere Polymernetzwerke erhalten, d.h. Netzwerke, die dadurch charakterisiert sind, dass sie eine engere Verteilung der Molmasse zwischen den Vernetzungspunkten aufweisen. Dies hat den Vorteil, dass Kettenspannungen durch Relaxationsprozesse besser abgebaut werden können. Dementsprechend lässt sich auch die Polymerisationsschrumpfungsspannung während der Aushärtung der Materialien verringern, was für eine dentale Anwendung z.B. als Füllungsmaterial ein großer Vorteil ist.

Die Silane der Formel I verfügen über radikalisch polymerisierbare Doppelbindungen, über die sie mit radikalisch polymerisierbaren Monomeren reagieren können, so dass sie bei der Härtung in das Polymernetzwerk eingebunden werden. Außerdem sind die Kondensate und die oberflächenmodifizierten Füllstoffe praktisch nicht in Wasser löslich, so dass sie beispielsweise durch Speichel nicht aus den gehärteten Materialien ausgewaschen werden.

Die Silane der Formel I werden vorzugsweise in Form ihrer Kondensate und in an Füllstoff gebundenere Form eingesetzt. Sie können aber auch als solche direkt zur Herstellung von radikalisch polymerisierbaren Werkstoffen verwendet werden. Die Silangruppen ermöglichen in diesem Fall *in situ* eine Anbindung an vorhandene Füllstoffe.

Die erfindungsgemäßen Silane, Kondensate und Füllstoffe eignen sich zur Herstellung von Polymerisaten, Kunststoffen, Duromeren und Verbundwerkstoffen/Kompositen u.a. für technische Anwendungen, zur Herstellen von Medizinprodukten, beispielsweise für die Chirurgie oder Augenheilkunde, und insbesondere zur Herstellung von Dentalwerkstoffen.

Hierzu können die Silane der Formel I und insbesondere deren Kondensate oder damit oberflächenmodifizierte Füllstoffe mit verschiedenartigen radikalisch polymerisierbaren Monomeren kombiniert werden. Besonders bevorzugt sind Werkstoffe, die als radikalisch polymerisierbares Monomer mindestens ein mono- oder multifunktionelles (Meth)acrylat enthalten. Unter monofunktionellen (Meth)acrylaten werden Verbindungen mit einer, unter polyfunktionellen (Meth)¬acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten. Materialien, die als radikalisch polymerisierbares Monomer mono- und multifunktionelle (Meth)acrylate enthalten, eignen sich besonders als Dentalwerkstoffe, wobei für Materialien, die intraoral gehärtet werden, Methacrylate bevorzugt sind.

Beispiele für besonders geeignete mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumylphenoxyethylenglycolmethacrylat (CMP-1E), Bisphenol-A-di(meth)acrylat, Bis-G(M)A (ein Additionsprodukt aus (Meth)acrylsäure und Bisphenol-A-diglycidylether), ethoxy- oder propoxyliertes Bisphenol-A-di(meth)-acrylat, wie z.B. das Bisphenol-A-dimethacrylat SR-348c (Sartomer) mit 3 Ethoxygruppen, oder 2,2-Bis[4-(2-(meth)acryloxypropoxy)phenyl]propan, UD(M)A (ein Additionsprodukt aus 2-Hydroxyethyl(meth)acrylat und 2,2,4-Trimethylhexamethylendiisocyanat), Di-, Tri- oder Tetraethylenglycoldi(meth)acrylat, Trimethylolpropantri-(meth)acrylat, Pentaerythrittetra(meth)acrylat, sowie Glycerindi- und tri(meth)acrylat, 1,4-Butandioldi(meth)acrylat, 1,10-Decandioldi(meth)acrylat (D₃MA) oder 1,12-Dodecandioldi(meth)acrylat.

Darüber hinaus enthalten die erfindungsgemäßen Werkstoffe vorzugsweise auch mindestens einen Initiator für die radikalische Polymerisation. Zur Initiierung der radikalischen Photopolymerisation werden vorzugsweise Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil, eingesetzt. Besonders bevorzugt werden Campherchinon (CQ) und 2,2-Dimethoxy-2-phenyl-acetophenon und ganz besonders bevorzugt α-Diketone in Kombination mit Aminen als Reduktionsmittel, wie z.B. 4-(Dimethylamino)-benzoesäureethylester (EDMAB), N,N-Dimethylaminoethylmethacrylat, N,N-Dimethyl-sym.-xylidin oder Triethanolamin, verwendet. Geeignet sind auch Norrish-Typ-I-Photoinitiatoren, vor allem Acyl- der Bisacylphosphinoxide, besonders geeignet sind Monoacyltrialkyl- oder Diacyldialkylgermanium-Verbindungen, wie z.B. Benzoyltrimethylgermanium, Dibenzoyldiethylgermanium oder Bis(4-methoxybenzoyl)diethylgermanium (MBDEGe). Dabei lassen sich vorteilhaft auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Zur Initiierung der radikalischen Polymerisation eignen sich besonders Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, *tert*-Butylperoctoat, *tert*-Butylperbenzoat oder Di-(*tert*-butyl)-peroxid. Zur Beschleunigung der Initiierung mittels Peroxiden lassen sich auch Kombinationen mit aromatischen Aminen einsetzen. Bevorzugte Redoxsysteme sind Kombinationen aus Benzoylperoxid mit Aminen, wie N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme bestehend aus Peroxiden und solchen Reduktionsmitteln, wie z.B. Ascorbinsäure, Barbiturate oder Sulfinsäuren oder Kombinationen von Hydroperoxiden mit Reduktionsmitteln und katalytischen Metallionen, wie, z.B. eine Mischung von Cumolhydroperoxid, einem Thioharnstoffderivat und Kupfer(II)-acetylacetonat, für die duale Aushärtung geeignet.

Gemäß einer bevorzugten Ausführungsform enthalten die erfindungsgemäßen Werkstoffe zusätzlich organischen oder vorzugsweise anorganischen partikulären Füllstoff, besonders bevorzugt einen oder mehrere anorganische partikuläre Füllstoffe. Mischungen, die Monomere und Füllstoffe enthalten, werden als Komposite bezeichnet.

Besonders geeignet sind Füllstoffe auf der Basis von Oxiden mit einer Partikelgröße von 0,010 bis 15 µm, wie SiO₂, ZrO₂ und TiO₂ bzw. Mischoxiden aus SiO₂, ZrO₂, ZnO und/oder TiO₂, nanopartikuläre oder mikrofeine Füllstoffe mit einer Partikelgröße von 10 bis 300 nm, wie pyrogene Kieselsäure oder Fällungskieselsäure sowie Glaspulver mit einer Partikelgröße von 0,01 bis 15 µm, vorzugweise von 0,2 bis 1,5 µm, wie Quarz-, Glaskeramik- oder röntgenopake Glaspulver von z.B. Barium- oder Strontiumaluminiumsilikatgläsern, und röntgenopake Füllstoffe mit einer Partikelgröße von 0,2 bis 5 µm, wie Ytterbiumtrifluorid, Tantal(V)-oxid, Bariumsulfat oder Mischoxide von SiO₂ mit Ytterbium(III)-oxid oder Tantal(V)-oxid. Faserförmige Füllstoffe, wie Nanofasern oder Whiskers können ebenfalls eingesetzt werden.

Wenn nicht anders angegeben handelt es sich hierin bei allen Partikelgrößen um gewichtsmittlere Partikelgrößen.

Die Füllstoffe werden nach der Partikelgröße unterteilt in Makrofüller und Mikrofüller. Makrofüller werden durch Mahlen von Quarz, röntgenopaken Gläsern, Borosilikaten oder von Keramik gewonnen, sind rein anorganischer Natur und bestehen meist aus splitterförmigen Teilchen. Bevorzugt sind Makrofüller mit einer mittleren Partikelgröße von 0,2 bis 10 µm. Als Mikrofüller werden vorzugsweise pyrogenes SiO₂ oder Fällungskieselsäure eingesetzt, oder auch Mischoxide, z.B. SiO₂-ZrO₂, die durch hydrolytische Cokondensation von Metallalkoxiden zugänglich sind. Die Mikrofüller haben vorzugsweise eine mittlere Partikelgröße von ca. 5 bis 100 nm.

Zur Verbesserung des Verbundes zwischen den Füllstoffpartikeln und der vernetzten Polymerisationsmatrix können SiO₂-basierende Füllstoffe mit (Meth)acrylatfunktionalisierten Silanen oberflächenmodifiziert werden. Ein Beispiel für solche Silane ist 3-(Meth)acryloyloxypropyltrimethoxysilan. Zur Oberflächenmodifizierung von nichtsilikatischen Füllstoffen, z.B. von ZrO₂ oder TiO₂, können auch funktionalisierte saure Phosphate, wie z.B. 10-(Meth)acryloyloxydecyldihydrogenphosphat eingesetzt werden.

Gegebenenfalls können die erfindungsgemäß eingesetzten Zusammensetzungen weitere Additive enthalten, vor allem Stabilisatoren, Farbmittel, mikrobiozide Wirkstoffe, fluoridionenabgebende Additive, Treibmittel, optische Aufheller, Weichmacher oder UV-Absorber.

Die erfindungsgemäßen Werkstoffe enthalten vorzugsweise die folgenden Komponenten:
(a) 1 bis 50 Gew.-%, bevorzugt 2 bis 40 Gew.-% und besonders bevorzugt 3 bis 30 Gew.-% mindestens eines Silans der Formel I, vorzugsweise mindestens eines Kondensates von einem oder mehreren Silanen der allgemeinen Formeln (I) und/oder mindestens eines Füllstoffs, der mit mindestens einem Silan der Formel I oberflächenmodifiziert ist,
(b) 0,01 bis 5 Gew.-%, bevorzugt 0,1 bis 5 Gew.-% und besonders bevorzugt 1,0 bis 3,0 Gew.-% mindestens eines Initiators für die radikalische Polymerisation,
(c) 5 bis 80 Gew.-%, bevorzugt 10 bis 60 Gew.-% und besonders bevorzugt 10 bis 50 Gew.-% mindestens eines radikalisch polymerisierbaren Monomers.
   Darüber hinaus enthalten die Werksoffe vorzugsweise auch
(d) 1 bis 80 Gew.-% mindestens eines von Komponente (a) verschiedenen Füllstoffs.

Der Füllungsgrad richtet sich nach dem gewünschten Anwendungszweck des Werkstoffs. Füllungskomposite haben vorzugsweise einen Füllstoffgehalt von 50-80 Gew.-% und Kompositzemente von 10-70 Gew.-%. Wenn als Komponente (a) ein füllstoffgebundenes Silan der Formel (I) eingesetzt wird, beziehen sich diese Mengenangaben auf die Gesamtmenge von (a) und (d).

Außerdem können die Werkstoffe vorteilhaft
(e) 0 bis 5 Gew.-%, bevorzugt 0 bis 3 Gew.-% und besonders bevorzugt 0,2 bis 3 Gew.-% Additiv(e) enthalten.

Wenn nicht anders angegeben beziehen sich alle Mengenangaben hierin auf die Gesamtmasse der Materialien. Die bevorzugten Mengenbereiche können jeweils separat gewählt werden.

Besonders bevorzugt sind Werkstoffe, die aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind. Besonders bevorzugt sind außerdem Werkstoffe, die neben den Silanen der Formel I, darauf basierenden Kondensaten oder damit oberflächenmodifizierten Füllstoffen keine anderen Kettenregler enthalten.

Die erfindungsgemäßen Werkstoffe eignen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken. Sie zeichnen sich dadurch aus, dass die Silane der Formel I nach der Härtung einerseits über eine radikalisch polymerisierbare Gruppe in das Polymernetzwerk eingebunden und andererseits über eine Silangruppe an einen Füllstoff gebunden oder in ein Kondensat integriert sind, so dass die Silane nicht aus dem gehärteten Material entweichen und insbesondere bei Kontakt mit Speichel nicht ausgewaschen werden. Außerdem weisen die Werkstoffe eine verringerte Polymerisationsschrumpfungsspannung (PKS) und eine verbesserte Schlagzähigkeit auf.

Die Dentalwerkstoffe eignen sich primär zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch extraoral eingesetzt werden, beispielsweise bei der Herstellung oder Reparatur von Dentalrestaurationen, wie Prothesen, künstlichen Zähnen, Inlays, Onlays, Kronen und Brücken.

Ein weiterer Gegenstand der Erfindung sind Homo- und Copolymerisate, die durch Polymerisation erfindungsgemäßer Dentalwerkstoffe erhalten werden können. Derartige Polymerisate lassen sich beispielsweise durch spanabhebende Verfahren zu Prothesen oder künstlichen Zähnen verarbeiten. Sie liegen vorzugsweise in Form von zylinderförmigen oder scheibenförmigen Rohlingen vor.

Die erfindungsgemäßen Materialien eignen sich außerdem zur Herstellung von Formkörpern, welche z.B. mittels Gießen, Pressen oder 3D Druck hergestellt werden können. Besonders die verbesserte Schlagzähigkeit lässt diese Materialien auf gleiches Niveau wie gängigen Thermoplasten kommen. Zusätzlich ist die geringe Verzögerung bei der Härtung für den 3D Druck vorteilhaft. Die erfindungsgemäßen Materialien können daher sehr gut durch Stereolithographie oder 3D-Druck verarbeitet werden.

Außerdem betrifft die Erfindung die Verwendung von Silanen der Formel I, Kondensaten davon und von damit oberflächenmodifizierten Füllstoffen als Kettenüberträger bei der radikalischen Polymerisation oder zur Kontrolle oder Steuerung der Netzwerkstruktur bei der radikalischen Polymerisation insbesondere von (Meth)acrylaten.

Die Erfindung wird im Folgenden anhand von Beispielen näher erläutert.

### Ausführungsbeispiele

### Beispiel 1:

### Synthese des Silans 2-(Toluol-4-sulfonyloxy)-acrylsäure-2-[N-(3-triethoxysilyl)-propylcarbamoyloxyethyl]ester

### 1. Stufe: Brenztraubensäure-2-(tetrahydropyran-2-yloxy)-ethylester

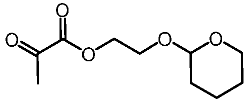

Eine Lösung von Brenztraubensäure (17.61 g, 0.20 mol), 2-(Tetrahydro-2H-pyran-2-yloxy)ethanol (29.24 g, 0.20 mol) und *N,N*-Dimethylaminopyridin (0.60 g, 5.0 mmol) in Dichlormethan (200 ml) wurde bei -5 °C portionsweise mit *N,N*'-Dicyclohexylcarbodiimid (45.39 g, 0.22 mol) versetzt. Die Suspension wurde 2 h bei -5 °C und anschließend bei Umgebungstemperatur gerührt. Nach 20 h wurde das Reaktionsgemisch mit *n*-Heptan (200 ml) verdünnt und über Kieselgel filtriert. Das Filtrat wurde am Rotationsverdampfer eingeengt und das Rohprodukt wurde mittels Säulenchromatographie (SiO₂, *n*-Heptan/Ethylacetat 2:1) gereinigt. Man erhielt 32.78 g (0.152 mol; 76%) einer gelblichen Flüssigkeit.
¹H-NMR (CDCl₃, 400 MHz): δ = 1.47-1.65 (m, 4H; CH₂-CH₂-CH₂), 1.66-1.87 (m, 2H; CH₂-CH₂-CH₂), 2.48 (s, 3H; CH₃), 3.48-3.56 (m, 1H; O-CH₂), 3.70-3.78 (m, 1H; O-CH₂), 3.81-3.89 (m, 1H; O-CH₂), 3.95-4.02 (m, 1H; O-CH₂), 4.38-4.49 (m, 2H; COO-CH₂), 4.66 (t, 1H; *J* = 3.3 Hz; O-CH-O).
¹³C-NMR (CDCl₃, 100.6 MHz): δ = 19.0 (CH₂), 25.2 (CH₂), 26.6 (CH₃), 30.2 (CH₂), 61.9 (O-CH₂), 64.4 (O-CH₂), 65.2 (O-CH₂), 98.5 (O-CH-O), 160.6 (C=O), 191.4 (C=O).
IR (rein): 2943 (w), 2872 (w), 1730 (vs), 1442 (w), 1385 (w), 1357 (w), 1299 (m), 1202 (w), 1184 (w), 1124 (vs), 1077 (s), 1033 (s), 1020 (s), 907 (w), 894 (w), 871 (m), 813 (m), 718 (w) cm⁻¹.
Anal. ber. für C₁₀H₁₆O₅: C, 55.55; H, 7.46. Gef.: C, 55.89; H, 7.25.

### 2. Stufe: 2-(Toluol-4-sulfonyloxy)-acrylsäure-2-(tetrahydropyran-2-yloxy)-ethylester

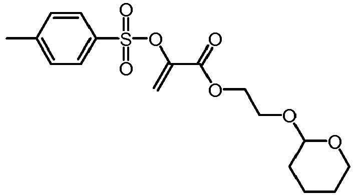

Zu einer Lösung von Brenztraubensäure-2-(tetrahydropyran-2-yloxy)-ethylester (32.58 g, 0.151 mol), *p*-Toluolsulfonylchlorid (28.72 g, 0.151 mol) und *N,N*-Dimethylaminopyridin (0.90 g, 7.5 mmol) in Dichlormethan (250 ml) wurde Triethylamin (27.45 g, 0.271 mol) zugetropft. Das Reaktionsgemisch wurde 20 h bei Umgebungstemperatur gerührt, mit Wasser (3x 100 ml) und gesättigter wässriger NaCl-Lösung (100 ml) gewaschen, über wasserfreiem Natriumsulfat getrocknet, filtriert und am Rotationsverdampfer eingeengt. Das Rohprodukt wurde mittels Säulenchromatographie (SiO₂, *n*-Heptan/Ethylacetat 4:1) gereinigt. Man erhielt 24.26 g (65.5 mmol; 43%) eines gelblichen Öls.
¹H-NMR (CDCl₃, 400 MHz): δ = 1.47-1.86 (m, 6H; CH₂-CH₂-CH₂), 2.46 (s, 3H; CH₃), 3.48-3.55 (m, 1H; O-CH₂), 3.59-3.65 (m, 1H; O-CH₂), 3.83-3.91 (m, 2H; O-CH₂), 4.22-4.33 (m, 2H; O-CH₂), 4.63 (t, 1H; *J* = 3.3 Hz; O-CH-O), 5.64 (d, 1H; *J* = 2.2 Hz; =CH), 6.16 (d, 1H; *J* = 2.2 Hz; =CH), 7.36 (d, 2H; *J* = 8.2 Hz; Ar-H), 8.85 (d, 2H; *J* = 8.2 Hz; Ar-H).
¹³C-NMR (CDCl₃, 100.6 MHz): δ = 19.1 (CH₂), 21.6 (CH₃), 25.2 (CH₂), 30.3 (CH₂), 61.9 (O-CH₂), 64.5 (O-CH₂), 64.9 (O-CH₂), 98.6 (O-CH-O), 117.0 (=CH₂), 128.5 (Ar-CH), 129.6 (Ar-CH), 132.4 (Ar-C), 142.9 (=C), 145.5 (Ar-C), 160.8 (C=O).
IR (rein): 2943 (w), 2871 (w), 1736 (m), 1639 (w), 1597 (w), 1494 (w), 1453 (w), 1378 (m), 1295 (m), 1194 (s), 1179 (s), 1151 (s), 1125 (vs), 1091 (s), 1078 (s), 1035 (m), 1019 (m), 985 (m), 958 (s), 905 (m), 872 (m), 814 (s), 780 (m), 711 (vs), 696 (s), 661 (s) cm⁻¹.
Anal. ber. für C₁₇H₂₂O₇S: C, 55.12; H, 5.99; S, 8.66. Gef.: C, 55.73; H, 6.06; S, 8.39.

### 3. Stufe: 2-(Toluol-4-sulfonyloxy)-acrylsäure-2-hydroxyethylester

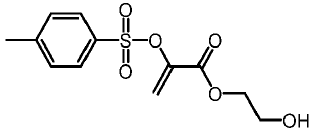

Eine Lösung von 2-(Toluol-4-sulfonyloxy)-acrylsäure-2-(tetrahydropyran-2-yloxy)-ethylester (24.16 g, 65.2 mmol) in Methanol (250 ml) wurde mit Amberlyst® 15 hydrogen form (10.0 g) versetzt und bei Umgebungstemperatur gerührt. Nach 20 h wurde das Reaktionsgemisch filtriert und das Filtrat am Rotationsverdampfer eingeengt. Das Rohprodukt wurde mittels Säulenchromatographie (SiO₂, Dichlormethan/Ethylacetat 4:1) gereinigt. Man erhielt 10.57 g (36.9 mmol; 57%) eines gelblichen Öls.
¹H-NMR (CDCl₃, 400 MHz): δ = 2.46 (s, 3H; CH₃), 2.76 (t, 1H; *J* = 6.5 Hz; OH), 3.78-3.84 (m, 2H; O-CH₂), 4.25-4.30 (m, 2H; O-CH₂), 5.50 (d, 1H; *J* = 2.3 Hz; =CH), 6.18 (d, 1H; *J* = 2.3 Hz; =CH), 7.37 (d, 2H; *J* = 8.2 Hz; Ar-H), 8.83 (d, 2H; *J* = 8.2 Hz; Ar-H).
¹³C-NMR (CDCl₃, 100.6 MHz): δ = 21.6 (CH₃), 60.3 (O-CH₂), 67.4 (O-CH₂), 117.5 (=CH₂), 128.3 (Ar-CH), 129.8 (Ar-CH), 132.0 (Ar-C), 142.9 (=C), 145.8 (Ar-C), 161.0 (C=O).
IR (rein): 3545 (br w), 1735 (s), 1639 (m), 1596 (m), 1494 (w), 1373 (s), 1294 (s), 1194 (s), 1178 (s), 1144 (vs), 1089 (s), 1018 (w), 956 (s), 918 (m), 877 (m), 815 (s), 781 (s) 710 (vs), 695 (s), 660 (s) cm⁻¹.
Anal. ber. für C₁₂H₁₄O₆S: C, 50.34; H, 4.93; S, 11.20. Gef.: C, 50.37; H, 4.97; S, 11.09.

### 4. Stufe: 2-(Toluol-4-sulfonyloxy)-acrylsäure-2-[N-(3-triethoxysilyl)-propylcarbamoyloxyethyl]ester

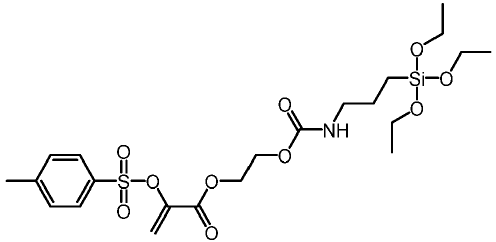

Zu einer Lösung von 2-(Toluol-4-sulfonyloxy)-acrylsäure-2-hydroxyethylester (10.47 g, 36.6 mmol) und Dibutylzinndilaureat (0.13 g, 0.2 mmol) in Aceton (20 ml) wurde 3-(Triethoxysilyl)propylisocyanat (9.04 g, 36.6 mmol) zugetropft. Die Reaktionslösung wurde bei Umgebungstemperatur gerührt und nach 24 h am Rotationsverdampfer eingeengt. Das Rohprodukt wurde mittels Säulenchromatographie (SiO₂, *n*-Heptan/Ethylacetat 1:1) gereinigt. Man erhielt 11.56 g (21.1 mmol; 58%) eines gelblichen Öls.
¹H-NMR (CDCl₃, 400 MHz): δ = 0.58-0.68 (m, 2H; Si-CH₂), 1.22 (t, 9H; *J* = 7.0 Hz; CH₂-CH₃), 1.57-1.68 (m, 2H; CH₂-CH₂-CH₂), 2.46 (s, 3H; CH₃), 3.11-3.22 (m, 2H; O-CH₂), 3.82 (q, 6H; *J* = 7.0 Hz; CH₂-CH₃), 4.17-4.24 (m, 2H; O-CH₂), 4.25-4.32 (m, 2H; O-CH₂), 5.15 - 5.30 (m, 1H; NH), 5.65 (d, 1H; *J* = 2.5 Hz; =CH), 6.15 (d, 1H; *J* = 2.5 Hz; =CH), 7.37 (d, 2H; *J* = 8.4 Hz; Ar-H), 8.84 (d, 2H; *J* = 8.4 Hz; Ar-H).
¹³C-NMR (CDCl₃, 100.6 MHz): δ = 7.4 (Si-CH₂), 18.0 (CH₃), 21.5 (CH₃), 23.0 (CH₂), 43.2 (CH₂), 58.2 (O-CH₂), 61.6 (O-CH₂), 63.9 (O-CH₂), 117.3 (=CH₂), 128.3 (Ar-CH), 129.6 (Ar-CH), 132.3 (Ar-C), 142.6 (=C), 145.5 (Ar-C), 155.8 (C=O), 160.5 (C=O).
²⁹Si-NMR (CDCl₃, 79.5 MHz): δ = -45.7.
IR (rein): 3339 (br w), 2974 (w), 2928 (w), 2887 (w), 2928 (w), 1727 (s), 1639 (w), 1597 (w), 1524 (w), 1444 (w), 1380 (m), 1296 (m), 1241 (m), 1180 (s), 1195 (s), 1156 (s), 1076 (vs), 957 (s), 815 (m), 779 (m), 713 (m), 696 (w), 663 (w) cm⁻¹.
Anal. ber. für C₂₂H₃₅NO₁₀SSi: C, 49.51; H, 6.61; N, 2.62; S, 6.01. Gef.: C, 50.44; H, 6.72; N, 2.95; S, 5.80.

### Beispiel 2:

### Hydrolytische Kondensation von 2-(Toluol-4-sulfonyloxy)-acrylsäure-2-[N-(3-triethoxysilyl)-propylcarbamoyloxyethyl]ester

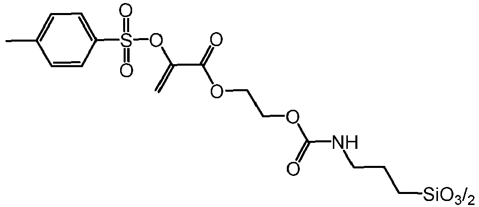

Eine Lösung von 2-(Toluol-4-sulfonyloxy)-acrylsäure-2-[*N*-(3-triethoxysilyl)-propylcarbamoyloxyethyl]ester (5.34 g, 10.0 mmol) in Ethanol (48.0 g) wurde mit Salzsäure (0.5 *M,* 0.54 g, 55.8 mmol) versetzt und 72 h bei Raumtemperatur gerührt. Das Lösungsmittel wurde entfernt und der Rückstand wurde bei 60 °C am Feinvakuum getrocknet. Man erhielt 4.09 g (9.6 mmol; 96 %) eines hochviskosen gelblichen Öls, das in Wasser unlöslich ist.
¹H-NMR (CDCl₃, 400 MHz): δ = 0.50-0.77 (m, 2H; Si-CH₂), 1.50-1.73 (m, 2H; CH₂-CH₂-CH₂), 2.45 (s, 3H; CH₃), 3.03-3.24 (m, 2H; O-CH₂), 4.15-4.38 (m, 4H; O-CH₂), 5.49-5.54 (m, 1H; =CH), 6.06-6.18 (m, 1H; =CH), 7.34 (d, 2H; *J* = 7.8 Hz; Ar-H), 7.82 (d, 2H; *J* = 7.8 Hz; Ar-H).

### Beispiel 3:

### Synthese des Silans 3-(Triethoxysilyl)-propyl-2-(tosylmethyl)-acrylat

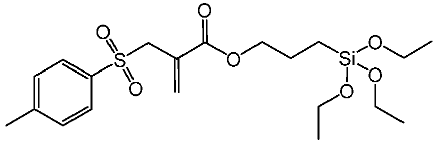

Unter Stickstoff wurde Diisopropylethylamin (23.4 mL, 0.134 mol) zu einer Lösung von 2-(Tosylmethyl)acrylsäure (29.36 g. 0.122 mol) und (3-lodpropyl)triethoxysilan (40.60 g. 0.122 mol) in trocknem Acetonitril (200 mL) zugetropft. Die Reaktionsmischung wurde 24 h bei 80 °C gerührt und dann im Vakuum eingeengt. Nach Zugabe von Diethylether (200 mL) fällt das Salz aus, das man abfiltriert. Das verbliebene Filtrat konzentriert man unter reduziertem Druck ein und reinigt den Rückstand mittels Säulenchromatographie (Eluent = Ethylacetat/Heptan: 1/1). Man erhält 34.04 g (63 % Ausbeute) eines farblosen Öls.
¹H NMR (CDCl₃, 400 MHz): δ = 0.53-0.62 (m, 2H, CH₂Si); 1.21 (t, ³J_{HH} = 7.0 Hz, 9H, OCH₂CH₃); 1.61-1.71 (m, 2H, CH₂CH₂Si); 2.42 (s, 3H, C_{Ar}CH₃); 3.80 (q, ³J_{HH} = 7.0 Hz, 6H, OCH₂CH₃); 3.92 (t, ³J_{HH} = 6.9 Hz, 2H, CH₂OCO); 4.11 (s, 2H, CH₂S); 5.87 (s, 1H, C=CH₂); 6.47 (s, 1H, C=CH₂); 7.28-7.33 (m, 2H, CH_{Ar}); 7.68-7.73 (m, 2H, CH_{Ar}). ¹³C NMR (CDCl₃, 101 MHz): δ = 6.5 (CH₂Si); 18.3 (OCH₂CH₃); 21.6 (C_{Ar}CH₃); 22.0 (CH₂CH₂Si); 57.5 (CH₂S); 58.4 (OCH₂CH₃); 67.5 (CH₂OCO); 128.7 (CH_{Ar}); 129.2 (C=CH₂); 129.6 (CH_{Ar}); 133.1 (C=CH₂); 135.5 (C_{Ar}); 144.9 (C_{Ar}); 164.8 (C=O). ²⁹Si NMR (CDCl₃, 79 MHz): -46.2.

### Beispiel 4:

### Hydrolytische Kondensation von 3-(Triethoxysilyl)-propyl-2-(tosylmethyl)acrylat

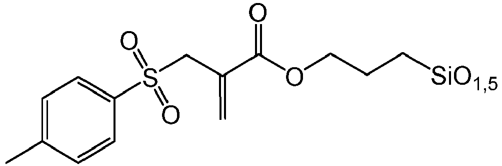

Eine Lösung von 3-(Triethoxysilyl)-propyl-2-(tosylmethyl)acrylat (8,91 g, 20.0 mmol) in Ethanol (110 ml) wurde mit Salzsäure (0.55 *M,* 1,07 g, 60 mmol) versetzt und 72 h bei Umgebungstemperatur gerührt. Das Lösungsmittel wurde entfernt und der Rückstand wurde bei 60 °C am Feinvakuum getrocknet. Man erhielt 6.35 g (95 % Ausbeute) eines hochviskosen gelblichen Öls, das in Wasser unlöslich ist.
¹H-NMR (CDCl₃, 400 MHz): δ = 0.68-0.77 (m, 2H; Si-CH₂), 1.61-1.74 (m, 2H; CH₂-CH₂-CH₂), 2.43 (s, 3H; CH₃), 3.79-3.82 (m, 2H; O-CH₂), 4.04-4.12 (m, 4H; SO₂-O-CH₂), 5.73-5.83 (m, 1H; =CH), 6.46 (s, 1H; =CH), 7.32 (d, 2H; Ar-H), 7.72 (d, 2H; Ar-H).

### Beispiel 5:

### Silanisierung von pyrogener Kieselsäure mit dem Silan 3-(Triethoxysilyl)-propyl-2-(tosylmethyl)acrylat aus Beispiel 3

Zu einer Suspension von 10.0 g pyrogener Kieselsäure (spez. Oberfläche 35-65 m²/g, Aerosil® OX50, Fa. Evonik) in 100 mL Cyclohexan wurden langsam 2.31 g (5.20 mmol) 3-(Triethoxysilyl)-propyl-2-(tosylmethyl)acrylat und anschließend 0.332 g (5.61 mmol) *n*-Propylamin zugetropft. Die Suspension wurde 24 h bei 20°C gerührt und anschließend bei 3000 U min⁻¹ zentrifugiert. Nach dem Abdekantieren des Cyclohexans wurde der Bodensatz noch zweimal mit Cyclohexan gewaschen und jeweils zentrifugiert. Der erhaltene Feststoff wurde am Rotavapor bei 40°C/0.1 mbar für 48 h getrocknet und danach gesiebt (90 µm). Es wurden 7.08 g eines weißen Pulvers erhalten, das in Wasser unlöslich ist (Glührückstand: 90.6 %, Blindwert OX50: 99.1 %).

### Beispiel 6:

### Herstellung und Photopolymerisation von Methacrylatharzen mit dem Kondensat aus Beispiel 2

Es wurde Mischung aus dem Urethandimethacrylat RM-3 (Additionsprodukt aus 2 Mol 2-Hydroxyethylmethacrylat und 1 Mol 2,2,4-Trimethylhexamethylendiisocyanat) und Triethylengylcoldimethacrylat (TEGDMA) im Gewichtsverhältnis 1:2 hergestellt. Dazu wurden als Photoinitiator 0.3 Gew.-% Ivocerin® (Bis(4-methoxybenzoyl)diethylgermanium, Ivoclar Vivadent AG) und 5.0 Gew.-% AFCT-Reagenz gegeben. Als AFCT-Reagenz wurde das Kondensat aus Beispiel 2 sowie als Referenzvinylsulfonester 2-(Toluol-4-sulfonyloxy)acrylsäureethylester TSAEE verwendet. Von den Photopolymerisationsharzen wurden entsprechende Prüfkörper präpariert, die 2 mal 3 Minuten mit einer dentalen Lichtquelle (Spectramat®, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit und des Biege-E-Moduls nach 24 h Wasserlagerung der Prüfkörper. Dabei zeigte sich bei Verwendung des AFCT-Reagenzes als Kondensat eine Verbesserung der mechanischen Eigenschaften (siehe Tabelle 1).

**Tabelle 1: Photopolymerisat-Eigenschaften**

| **Photopolymerisat** | **Biegefestigkeit (MPa)** | **Biege-E-Modul (GPa)** |
|---|---|---|
| **A** (ohne AFCT-Reagenz)* | 81.0 | 1.82 |
| **B** (mit Kondensat Bsp. 2) | 91.5 | 2.07 |
| **C** (mit TSAEE)* | 83.9 | 1.91 |

| | | |
|---|---|---|
| * Vergleichsbeispiel | | |

Darüber hinaus wurden die Prüfkörper im Mörser zerrieben und die pulverförmigen Polymerisate in wässrigem Ethanol (50 Vol.-% Ethanol) dispergiert. Nach 72 h Rühren wurden die festen Anteile abgetrennt und im flüssigen Rückstand mittels HPLC untersucht. Dabei zeigte sich, dass aus dem Referenzpolymerisat **C** Anteile des AFCT-Reagenzes TSAEE herauswaschbar sind, während im Eluat der Probe **B** kein AFCT-Reagenz nachweisbar war.

### Beispiel 7:

### Herstellung und Photopolymerisation von Methacrylatharzen mit dem Kondensat aus Beispiel 4

Es wurde Mischung aus dem Urethandimethacrylat RM-3 und TEGDMA im Gewichtsverhältnis 1:2 hergestellt. Dazu wurden als Photoinitiator 0.3 Gew.-% Ivocerin® und 5 Gew.-% AFCT-Reagenz gegeben. Als AFCT-Reagenz wurde das Kondensat aus Beispiel 4 sowie als Referenzallylsulfon 2-(Toluol-4-sulfonylmethyl)acrylsäureethylester TSMAEE verwendet. Von den Photopolymerisationsharzen wurden entsprechende Prüfkörper präpariert, die 2 mal 3 Minuten mit einer dentalen Lichtquelle (Spectramat®, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit und des Biege-E-Moduls nach 24 h Wasserlagerung der Prüfkörper:

**Tabelle 2: Photopolymerisat-Eigenschaften**

| **Photo polymerisat** | **Biegefestigkeit (MPa)** | **Biege-E-Modul (GPa)** |
|---|---|---|
| **D** (ohne AFCT-Reagenz)* | 81.0 | 1.82 |
| **E** (mit Kondensat Bsp. 4) | 84.7 | 1.90 |
| **F** (mit TSMAEE)* | 73.0 | 1.68 |

| | | |
|---|---|---|
| * Vergleichsbeispiel | | |

Auch hier zeigte sich bei Verwendung des AFCT-Reagenzes als Kondensat eine Verbesserung der mechanischen Eigenschaften (siehe Tabelle 2).

Darüber hinaus wurden die Prüfkörper im Mörser zerrieben und die pulverförmigen Polymerisate in wässrigem Ethanol (50 Vol.-% Ethanol) dispergiert. Nach 72 h Rühren wurden die festen Anteile abgetrennt und im flüssigen Rückstand mittels HPLC untersucht. Dabei zeigte sich, dass aus dem Referenzpolymerisat **F** Anteile des AFCT-Reagenzes TSAEE herauswaschbar sind, während im Eluat der Probe **E** kein AFCT-Reagenz nachweisbar war.

## Patentansprüche

1. Radikalisch polymerisierbares Silan gemäß der allgemeinen Formel I 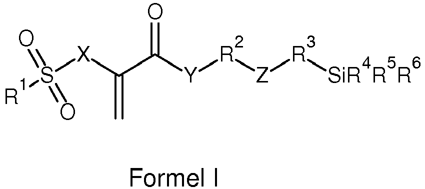 in der
R¹ ein linearer oder verzweigter aliphatischer C₁-C₉-Alkyl-Rest, Phenyl- oder alkylierter Phenyl-Rest ist,
R², R³ unabhängig voneinander jeweils entfallen oder ein linearer oder verzweigter aliphatischer C₁-C₂₀-Alkylen-Rest sind, der durch S- oder O-Atome unterbrochen sein kann,
R⁴, R⁵, R⁶ unabhängig voneinander jeweils -Cl, -O-CH₃, -O-C₂H₅, -CH₃ oder -C₂H₅ sind,
X CH₂ oder O ist,
Y entfällt, O oder NR', wobei R' H oder ein C₁₋₅-Alkylrest ist, und
Z entfällt, O, NR", -CO-O-, -CO-NR"-, -O-CO-O-, -O-CO-NR"-, oder -NR"-CO-NR"- ist, wobei R" H oder ein C₁₋₅-Alkylrest ist
und wobei die Reste R² und R³ nicht gleichzeitig entfallen können und Z entfällt, wenn R² oder R³ entfällt.

2. Radikalisch polymerisierbares Silan nach Anspruch 1, wobei
R¹ -CH₃, -C₂H₅, Phenyl oder Tolyl ist,
R², R³ unabhängig voneinander jeweils entfallen oder ein linearer aliphatischer C₁-C₁₀-Alkylen-Rest sind, der durch O-Atome unterbrochen sein kann,
R⁴, R⁵, R⁶ unabhängig voneinander jeweils -O-CH₃ oder -O-C₂H₅ sind,
X CH₂ oder O ist,
Y entfällt oder O ist, und
Z entfällt, O, -CO-O-, -CO-NH-, -O-CO-O- oder -O-CO-NH- ist.

3. Radikalisch polymerisierbares Silan nach Anspruch 1, wobei
R¹ -CH₃, -C₂H₅, Phenyl oder Tolyl ist,
R² ein linearer aliphatischer C₂-C₈-Alkylen-Rest ist, der durch O-Atome unterbrochen sein kann,
R³ entfällt oder ein linearer aliphatischer C₁-C₆-Alkylen-Rest ist,
R⁴, R⁵, R⁶ unabhängig voneinander jeweils -O-CH₃ oder -O-C₂H₅ sind,
X CH₂ oder O ist,
Y O ist, und
Z entfällt, -CO-NH- oder -O-CO-NH- ist.

4. Verfahren zur Herstellung von Polysiloxan-Kondensaten, bei dem man mindestens ein Silan gemäß einem der Ansprüche 1 bis 3 mit einer stöchiometrischen Menge oder einem Überschuss an Wasser versetzt und man dann die Mischung reagieren lässt.

5. Verfahren nach Anspruch 4, bei dem man das Silan zunächst in einem organischen Lösungsmittel löst, man die Lösung danach mit Wasser oder einer Mischung von Wasser und einem organischem Lösungsmittel versetzt, man die Mischung dann bei einer Temperatur im Bereich von 0°C bis 140°C reagieren lässt und man anschließend das Lösungsmittel und flüchtige Bestandteile entfernt.

6. Verfahren nach Anspruch 4 oder 5, bei dem man die Mischung zusätzlich mit einem Katalysator, vorzugsweise mit einer Säure, z.B. Essigsäure oder Salzsäure, einer Base, z.B. Ammoniak, Amine, NaOH, Methylimidazol, oder Ammoniumfluorid versetzt.

7. Verfahren zur Herstellung eines oberflächenmodifizierten Füllstoffs, bei dem man einen Füllstoff in einem organischen Lösungsmittel dispergiert, man die Dispersion mit mindestens einem Silan gemäß einem der Ansprüche 1 bis 3, Wasser und vorzugsweise einem Katalysator versetzt und rührt, und man anschließend den Füllstoff vom Lösungsmittel abtrennt.

8. Verfahren nach Anspruch 7, bei dem man als Füllstoff einen anorganischen partikulären Füllstoff, wie Quarzpulver, Barium- oder Strontium-Aluminiumsilikat-Glaspulver, einen röntgenopaken Füllstoff, wie Ytterbiumtrifluorid, einen amorphen kugelförmigen Füllstoff auf der Basis von Oxid, wie pyrogene Kieselsäure, Fällungskieselsäure, ZrO₂, ZnO, TiO₂ oder ein Mischoxid aus SiO₂, ZrO₂ und/oder TiO₂, partikuläres Tantal(V)-oxid, Bariumsulfat oder ein Mischoxid von SiO₂ mit Ytterbium(III)-oxid oder Tantal(V)-oxid, einen faserförmigen Füllstoff, wie Nanofasern, Glasfasern, Polyamid- oder Kohlenstoff-Fasern, einsetzt.

9. Verfahren nach Anspruch 7 oder 8, bei dem man die Mischung bei einer Temperatur im Bereich von 0 bis 100 °C für 1 bis 20 Stunden rührt.

10. Polysiloxan-Kondensat, das nach einem Verfahren gemäß einem der Ansprüche 4 bis 6 erhältlich ist.

11. Oberflächenmodifizierter Füllstoff, der nach einem Verfahren gemäß einem der Ansprüche 7 bis 9 erhältlich ist.

12. Dentalwerkstoff, **dadurch gekennzeichnet, dass** er ein Silan nach einem der Ansprüche 1 bis 4, ein Polysiloxan-Kondensat nach Anspruch 10 und/oder einen oberflächenmodifizierten Füllstoff nach Anspruch 11 enthält.

13. Dentalwerkstoff nach Anspruch 12, der
(a) 1 bis 50 Gew.-%, bevorzugt 2 bis 40 Gew.-% und besonders bevorzugt 3 bis 30 Gew.-% mindestens eines Silans der Formel I; mindestens eines Polysiloxan-Kondensats nach Anspruch 10 und/oder mindestens eines oberflächenmodifizierten Füllstoffs nach Anspruch 11,
(b) 0,01 bis 5 Gew.-%, bevorzugt 0,1 bis 5 Gew.-%, und besonders bevorzugt 1,0 bis 3,0 Gew.-% mindestens eines Initiators für die radikalische Polymerisation und
(c) 5 bis 80 Gew.-%, bevorzugt 10 bis 60 Gew.-% und besonders bevorzugt 10 bis 50 Gew.-% mindestens eines radikalisch polymerisierbaren Monomers enthält, jeweils bezogen auf die Gesamtmasse des Werkstoffs.

14. Dentalwerkstoff nach Anspruch 13, der zusätzlich
(d) 1 bis 80 Gew.-%, bevorzugt 10-70 Gew.-% oder 50-80 Gew.-% Füllstoff enthält.

15. Verwendung eines Silans gemäß einem der Ansprüche 1 bis 3, eines Polysiloxan-Kondensats nach Anspruch 10 oder eines oberflächenmodifizierten Füllstoffs nach Anspruch 11 zur Herstellung eines Dentalwerkstoffs.

## Claims

1. Radically polymerisable silane according to the general Formula I 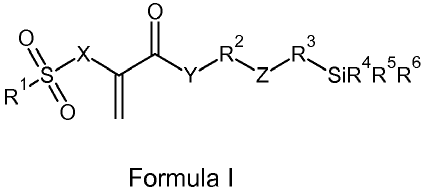 in which
R¹ is a linear or branched aliphatic C₁-C₉ alkyl group, phenyl or alkylated phenyl group,
R², R³ independently of each other in each case are absent or are a linear or branched aliphatic C₁-C₂₀ alkylene group that can be interrupted by S or O atoms,
R⁴, R⁵, R⁶ independently of each other in each case are -Cl, -OCH₃, -OC₂H₅, -CH₃ or -C₂H₅,
X is CH₂ or O,
Y is absent, or is O or NR', wherein R' is H or a C₁₋₅ alkyl group, and
Z is absent, or is O, NR", -CO-O-, -CO-NR"-, -O-CO-O-, -O-CO-NR"-, or -NR"-CO-NR"-, wherein R" is H or a C₁₋₅ alkyl group
and wherein the groups R² and R³ cannot be absent at the same time and Z is absent if R² or R³ is absent.

2. Radically polymerisable silane according to claim 1, wherein
R¹ is -CH₃, -C₂H₅, phenyl or tolyl,
R², R³ independently of each other in each case are absent or are a linear aliphatic C₁-C₁₀ alkylene group that can be interrupted by O atoms,
R⁴, R⁵, R⁶ independently of each other in each case are -OCH₃ or -OC₂H₅,
X is CH₂ or O,
Y is absent or is O, and
Z is absent, or is O, -CO-O-, -CO-NH-, -O-CO-O- or -O- CO-NH-.

3. Radically polymerisable silane according to claim 1, wherein
R¹ is -CH₃, -C₂H₅, phenyl or tolyl,
R² is a linear aliphatic C₂-C₈ alkylene group that can be interrupted by O atoms,
R³ is absent or is a linear aliphatic C₁-C₆ alkylene group,
R⁴, R⁵, R⁶ independently of each other in each case are -OCH₃ or -OC₂H₅,
X is CH₂ or O,
Y is O, and
Z is absent, or is -CO-NH- or -O-CO-NH-.

4. Process for the production of polysiloxane condensates, in which at least one silane according to one of claims 1 to 3 is mixed with a stoichiometric amount or an excess of water and the mixture is then allowed to react.

5. Process according to claim 4, in which the silane is initially dissolved in an organic solvent, the solution is then mixed with water or a mixture of water and an organic solvent, the mixture is then allowed to react at a temperature in the range of from 0 °C to 140 °C and the solvent and volatile components are subsequently removed.

6. Process according to claim 4 or 5, in which the mixture is additionally mixed with a catalyst, preferably with an acid, e.g. acetic acid or hydrochloric acid, a base, e.g. ammonia, amines, NaOH, methylimidazole, or ammonium fluoride.

7. Process for the production of a surface-modified filler, in which a filler is dispersed in an organic solvent, the dispersion is mixed with at least one silane according to one of claims 1 to 3, water and preferably a catalyst and stirred, and the filler is subsequently separated from the solvent.

8. Process according to claim 7, in which an inorganic particulate filler, such as quartz powder, barium or strontium aluminium silicate glass powder, an X-ray opaque filler, such as ytterbium trifluoride, an amorphous spherical filler based on oxide, such as fumed silica, precipitated silica, ZrO₂, ZnO, TiO₂ or a mixed oxide of SiO₂, ZrO₂ and/or TiO₂, particulate tantalum(V) oxide, barium sulfate or a mixed oxide of SiO₂ with ytterbium(III) oxide or tantalum(V) oxide, a fibrous filler, such as nanofibres, glass fibres, polyamide or carbon fibres, is used as the filler.

9. Process according to claim 7 or 8, in which the mixture is stirred at a temperature in the range of from 0 to 100 °C for 1 to 20 hours.

10. Polysiloxane condensate that can be obtained according to a process according to one of claims 4 to 6.

11. Surface-modified filler that can be obtained according to a process according to one of claims 7 to 9.

12. Dental material, **characterised in that** it comprises a silane according to one of claims 1 to 4, a polysiloxane condensate according to claim 10 and/or a surface-modified filler according to claim 11.

13. Dental material according to claim 12 which comprises
(a) 1 to 50 wt %, preferably 2 to 40 wt % and particularly preferably 3 to 30 wt % of at least one silane of Formula I; at least one polysiloxane condensate according to claim 10 and/or at least one surface-modified filler according to claim 11,
(b) 0.01 to 5 wt %, preferably 0.1 to 5 wt %, and particularly preferably 1.0 to 3.0 wt % of at least one initiator for the radical polymerisation and
(c) 5 to 80 wt %, preferably 10 to 60 wt % and particularly preferably 10 to 50 wt % of at least one radically polymerisable monomer, in each case based on the total mass of the material.

14. Dental material according to claim 13 additionally comprising
(d) 1 to 80 wt %, preferably 10-70 wt % or 50-80 wt % filler.

15. Use of a silane according to one of claims 1 to 3, a polysiloxane condensate according to claim 10 or a surface-modified filler according to claim 11 for the production of a dental material.

## Revendications

1. Silane polymérisable par voie radicalaire, de formule générale I : 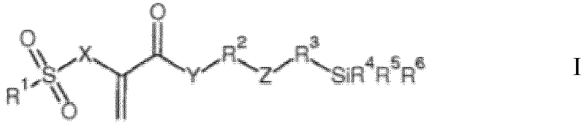 dans laquelle
- R¹ représente un groupe aliphatique alkyle en C₁-C₉, linéaire ou ramifié, un groupe phényle, ou un groupe phényle alkylé,
- R² et R³, chacun indépendamment l'un de l'autre, ne représentent rien ou représentent un groupe aliphatique alcanediyle en C₁-C₂₀, linéaire ou ramifié, qui peut être interrompu par un ou des atome(s) de soufre ou d'oxygène,
- R⁴, R⁵ et R⁶ représentent, chacun indépendamment des autres, une entité symbolisée par -Cl, -O-CH₃, -O-C₂H₅, -CH₃ ou -C₂H₅,
- X représente un chaînon symbolisé par CH₂ ou O,
- Y ne représente rien ou représente un chaînon symbolisé par O ou NR', où R' représente un atome d'hydrogène ou un groupe alkyle en C₁-C₅,
- et Z ne représente rien ou représente un chaînon ou fragment symbolisé par O, NR", -CO-O-, -CO-NR"-, -O-CO-O-, -O-CO-NR"- ou -NR"-CO-NR"-, où R" représente un atome d'hydrogène ou un groupe alkyle en C₁-C₅,
étant entendu que R² et R³ ne peuvent pas ne rien représenter en même temps, et que Z ne représente rien si R² ou R³ ne représente rien.

2. Silane polymérisable par voie radicalaire, conforme à la revendication 1, dans lequel
- R¹ représente un groupe méthyle, éthyle, phényle ou tolyle,
- R² et R³, chacun indépendamment l'un de l'autre, ne représentent rien ou représentent un groupe aliphatique alcanediyle en C₁-C₁₀ linéaire, qui peut être interrompu par un ou des atome(s) d'oxygène,
- R⁴, R⁵ et R⁶ représentent, chacun indépendamment des autres, un groupe méthoxy ou éthoxy,
- X représente un chaînon symbolisé par CH₂ ou O,
- Y ne représente rien ou représente un chaînon symbolisé par O,
- et Z ne représente rien ou représente un chaînon ou fragment symbolisé par O, -CO-O-, -CO-NH-, -O-CO-O- ou -O-CO-NH-.

3. Silane polymérisable par voie radicalaire, conforme à la revendication 1, dans lequel
- R¹ représente un groupe méthyle, éthyle, phényle ou tolyle,
- R² représente un groupe aliphatique alcanediyle en C₂-C₈ linéaire, qui peut être interrompu par un ou des atome(s) d'oxygène
- R³ ne représente rien ou représente un groupe aliphatique alcanediyle en C₁-C₆ linéaire,
- R⁴, R⁵ et R⁶ représentent, chacun indépendamment des autres, un groupe méthoxy ou éthoxy,
- X représente un chaînon symbolisé par CH₂ ou O,
- Y représente un chaînon symbolisé par O,
- et Z ne représente rien ou représente un fragment symbolisé par -CO-NH- ou -O-CO-NH-.

4. Procédé de préparation de condensats polysiloxanes, dans lequel on ajoute, à au moins un silane conforme à l'une des revendications 1 à 3, de l'eau en quantité stœchiométrique ou en excès, puis on laisse le mélange réagir.

5. Procédé conforme à la revendication 4, dans lequel on commence par dissoudre le silane dans un solvant organique, puis on ajoute à la solution de l'eau ou un mélange d'eau et d'un solvant organique, on laisse ensuite le mélange réagir à une température située dans l'intervalle allant de 0 à 140 °C, puis on élimine le solvant et les constituants volatils.

6. Procédé conforme à la revendication 4 ou 5, dans lequel on ajoute en plus au mélange un catalyseur, de préférence un acide, par exemple de l'acide acétique ou de l'acide chlorhydrique, une base, par exemple de l'ammoniac, une amine, de l'hydroxyde de sodium ou du méthyl-imidazole, ou du fluorure d'ammonium.

7. Procédé de préparation d'une charge modifiée en surface, dans lequel on disperse une charge dans un solvant organique, on ajoute à la dispersion au moins un silane conforme à l'une des revendications 1 à 3, de l'eau, et de préférence un catalyseur, et l'on agite le tout, puis on sépare la charge du solvant.

8. Procédé conforme à la revendication 7, dans lequel, en tant que charge, on utilise une charge inorganique en particules, comme du quartz en poudre, un verre de silicate d'aluminium et de baryum ou de strontium en poudre, une charge opaque aux rayons X comme du tri-fluorure d'ytterbium, une charge amorphe à base d'oxyde, en particules sphériques, comme de la silice de pyrohydrolyse, de la silice de précipitation, de la zircone, de l'oxyde de zinc, du dioxyde de titane, ou un oxyde mixte à base de silice, zircone et/ou dioxyde de titane, de l'oxyde de tantale-(V) en particules, du sulfate de baryum ou un oxyde mixte à base de silice et d'oxyde d'ytterbium-(III) ou d'oxyde de tantale-(V), ou une charge en fibres, comme des nanofibres, des fibres de verre, des fibres de polyamide ou des fibres de carbone.

9. Procédé conforme à la revendication 7 ou 8, dans lequel on agite le mélange durant 1 à 20 heures à une température située dans l'intervalle allant de 0 à 100 °C.

10. Condensat polysiloxane, accessible par un procédé conforme à l'une des revendications 4 à 6.

11. Charge modifiée en surface, accessible par un procédé conforme à l'une des revendications 7 à 9.

12. Matériau dentaire, **caractérisé en ce qu'**il contient un silane conforme à l'une des revendications 1 à 4, un condensat polysiloxane conforme à la revendication 10, ou une charge modifiée en surface, conforme à la revendication 11.

13. Matériau dentaire conforme à la revendication 12, qui contient :
a) de 1 à 50 % en poids, de préférence de 2 à 40 % en poids, et mieux encore de 3 à 30 % en poids, d'au moins un silane de formule I, d'au moins un condensat polysiloxane conforme à la revendication 10, et/ou d'au moins une charge modifiée en surface, conforme à la revendication 11,
b) de 0,01 à 5 % en poids, de préférence de 0,1 à 5 % en poids,
et mieux encore de 1,0 à 3,0 % en poids, d'au moins un amorceur de polymérisation par voie radicalaire,
c) et de 5 à 80 % en poids, de préférence de 10 à 60 % en poids,
et mieux encore de 10 à 50 % en poids, d'au moins un monomère polymérisable par voie radicalaire,
dans chaque cas par rapport au poids total du matériau.

14. Matériau dentaire conforme à la revendication 13, qui contient en outre :
d) de 1 à 80 % en poids, et de préférence de 10 à 70 % en poids ou de 50 à 80 % en poids, d'une charge.

15. Utilisation d'un silane conforme à l'une des revendications 1 à 3, d'un condensat polysiloxane conforme à la revendication 10, ou d'une charge modifiée en surface, conforme à la revendication 11, pour la préparation d'un matériau dentaire.
